# EUROPEAN PATENT APPLICATION

(11) **EP 2 489 669 A1**
(43) Date of publication of application: **22.08.2012**
(21) Application number: 11001353.9
(22) Date of filing: 18.02.2011
(51) Int. Cl.: C07D 498/04, A61K 31/407, A61P 31/12, C07K 5/00, A61K 38/03

(54) **Haprolid and derivatives thereof as inhibitors of HCV**

(71) Applicant: Helmholtz-Zentrum für Infektionsforschung GmbH, 38124 Braunschweig (DE); Twincore, 30625 Hannover (DE)
(72) Inventor: Pietschmann, Thomas, Prof. Dr., 30559 Hannover (DE); Gentzsch, Juliane, 30625 Hannover (DE); Steinmetz, Heinrich, 31139 Hildesheim (DE); Kunze, Brigitte, Dr., 38124 Braunschweig (DE)
(74) Representative: Forstmeyer, Dietmar

(57) **Abstract**

The present invention describes new compounds (e.g. Haprolid) of formula (I) and their use as antiviral agents such as inhibitors of hepatitis C virus (HCV):

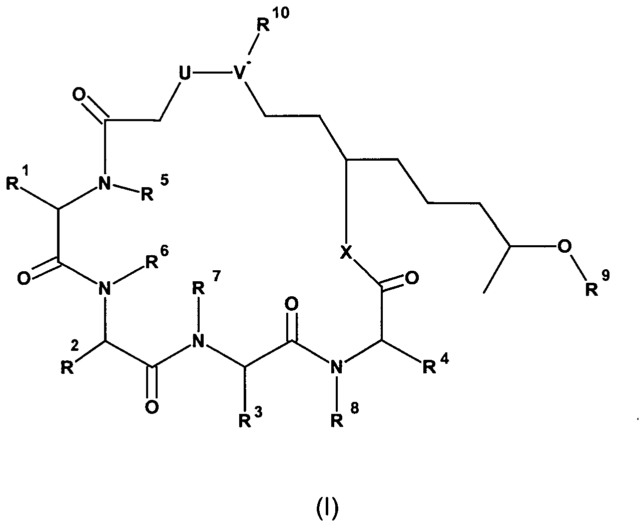

## Description

Hepatitis C virus (HCV) causes chronic infection associated with severe liver disease. About 130 million people are chronically infected worldwide. Current treatment with pegylated interferon-α and ribavirin has limited efficacy and is associated with substantial side effects. Although highly potent HCV-specific enzyme inhibitors are in clinical development, drug-resistance and genotype-specific differences in efficacy may limit these novel therapeutics. Therefore, a combination of drugs with distinct mechanisms of action targeting different steps of the viral replication cycle will likely improve viral response rates and therapy success. Furthermore, 25-30% of HIV-infected patients are also positive for HCV and would greatly benefit from an inhibitor that could target both viruses.

It has been an object of the present invention to provide novel antiviral compounds that are preferably inhibitors of hepatitis C virus (HCV).

The present invention relates to one or more compounds of general formula (I) and mixtures thereof: 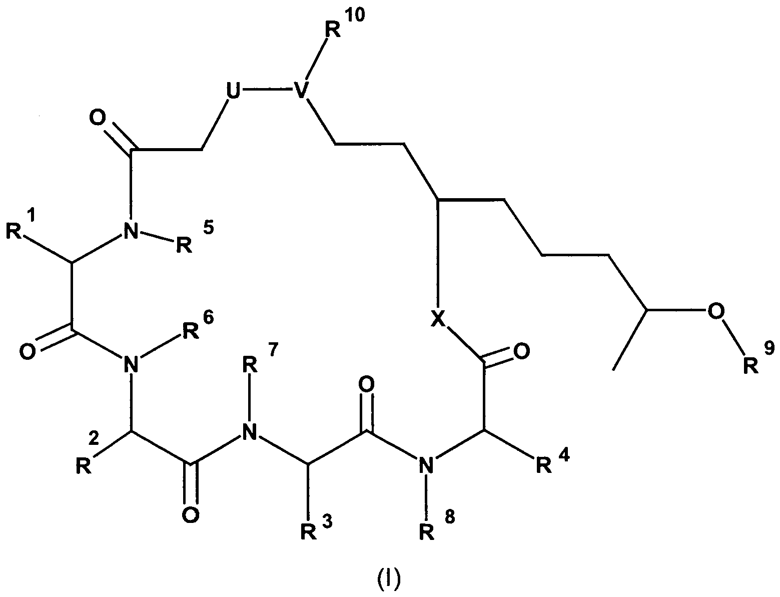 wherein
X is O, S or NR¹¹;
U-V are selected from the following groups: 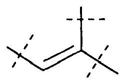 (cis or trans) and 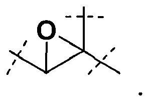 R¹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R² is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R³ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁴ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁵ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁶ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁷ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁸ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R¹⁰ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group; and
R¹¹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group; or
R¹ and R⁵ and/or R¹ and R⁶ and/or R⁵ and R⁶ and/or R¹ and R² and/or R² and R⁶ and/or R² and R⁷ and/or R⁶ and R⁷ and/or R² and R³ and/or R³ and R⁷ and/or R³ and R⁸ and/or R⁷ and R⁸ and/or R³ and R⁴ and/or R⁴ and R⁸ and/or R⁴ and R¹¹ and/or R⁸ and R¹¹ together are part of an optionally substituted heterocycloalkyl group;
or a pharmacologically acceptable salt, solvate, hydrate or a pharmacologically acceptable formulation thereof.

The expression alkyl refers to a saturated, straight-chain or branched hydrocarbon group that contains from 1 to 20 carbon atoms, preferably from 1 to 12 carbon atoms, especially from 1 to 6 carbon atoms, for example a methyl, ethyl, propyl, iso-propyl, n-butyl, iso-butyl, tert-butyl, n-pentyl, n-hexyl, 2,2-dimethylbutyl or n-octyl group.

The expressions alkenyl and alkynyl refer to at least partially unsaturated, straight-chain or branched hydrocarbon groups that contain from 2 to 20 carbon atoms, preferably from 2 to 12 carbon atoms, especially from 2 to 6 carbon atoms, for example an ethenyl, allyl, acetylenyl, propargyl, isoprenyl or hex-2-enyl group. Preferably, alkenyl groups have one or two (especially one) double bond(s) and alkynyl groups have one or two (especially one) triple bond(s).

The expression heteroalkyl refers to an alkyl, alkenyl (e.g. heteroalkenyl) or alkynyl group in which one or more (preferably 1, 2 or 3) carbon atoms have been replaced by an oxygen, nitrogen, phosphorus, boron, selenium, silicon or sulphur atom (preferably oxygen, sulphur or nitrogen). Examples for heteroalkyl groups are alkyloxy, alkyloxyalkyl, alkenyloxyalkyl, alkenyloxy, alkyloxyalkenyl, alkylamino, alkylaminoalkyl, dialkylamino, dialkylaminoalkyl, alkylthio or alkylthioalkyl groups. The expression heteroalkyl furthermore refers to a carboxylic acid or to a group derived from a carboxylic acid such as, for example, acyl, acyloxy, acyloxyalkyl, acylalkyl, alkoxycarbonyl, alkyloxycarbonylalkyl, carboxyalkylamide, alkylcarbonylamino, alkylcarbonylaminoalkyl, alkylaminocarbonyl, alkylaminocarbonylalkyl, or alkoxycarbonyloxy.

Examples of heteroalkyl groups are groups of formulae R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-R^{a}-S-CO-N(R^{b})-Y^{a}-, R^{a}N(R^{b})-CO-S-Y^{a}, R^{a}-S-CO-O-Y^{a}-, R^{a}-O-CO-S-Y^{a}-, R^{a}S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-(preferred are R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N(R b)-Y^{a}- and R^{a}-N(R^{b})-CO-Y^{a}-,), R^{a} being a hydrogen atom, a C₁-C₆alkyl, a C₂-C₆alkenyl or a C₂-C₆alkynyl group; R^{b} being a hydrogen atom, a C₁-C₆alkyl, a C₂-C₆alkenyl or a C₂-C₆alkynyl group; R^{c} being a hydrogen atom, a C₁-C₆alkyl, a C₂-C₆alkenyl or a C₂-C₆alkynyl group; R^{d} being a hydrogen atom, a C₁-C₆alkyl, a C₂-C₆alkenyl or a C₂-C₆alkynyl group and Y^{a} being a bond, a C₁-C₆alkylene, a C₂-C₆alkenylene or a C₂-C₆alkynylene group (preferrably, R^{a}, R^{b}, R^{c} and R^{d} are independently H or C₁-C₆ alkyl and Y^{a} is a bond or C₂-C₆alkylene), each heteroalkyl group containing at least one carbon atom and it being possible for one or more hydrogen atoms to have been replaced by fluorine or chlorine atoms. Specific examples of heteroalkyl groups are methoxy, trifluoromethoxy, ethoxy, n-propyloxy, isopropyloxy, *tert*-butyloxy, methoxymethyl, ethoxymethyl, methoxyethyl, methylamino, ethylamino, dimethylamino, diethylamino, isopropylethylamino, methylaminomethyl, ethylaminomethyl, diisopropylaminoethyl, enol ether, dimethylaminomethyl, dimethylaminoethyl, acetyl, propionyl, butyryloxy, acetyloxy, methoxycarbonyl, ethoxycarbonyl, N-ethyl-N-methylcarbamoyl and N-methylcarbamoyl. Further examples of heteroalkyl groups are nitrile, isonitrile, cyanate, thiocyanate, isocyanate, isothiocyanate and alkylnitrile groups.

Furthermore, the terms alkyl, alkenyl, alkynyl and heteroalkyl refer to groups in which one or more hydrogen atoms have been replaced by a halogen atom (preferably F or Cl) such as, for example, a 2,2,2-trichloroethyl or a trifluoromethyl or a trifluoromethyloxy group.

The expression cycloalkyl refers to a saturated or partially unsaturated (for example cycloalkenyl), cyclic group that contains one or more rings (preferably 1 or 2), containing from 3 to 50 carbon atoms, preferably 3 to 14, further preferably from 3 to 10 (especially 3, 4, 5, 6 or 7) carbon atoms. The expression cycloalkyl refers furthermore to groups in which one or more hydrogen atoms have been replaced by fluorine, chlorine, bromine or iodine atoms or by OH, =O, SH, =S, NH₂, =NH or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetralin, cyclopentylcyclohexyl, fluorocyclohexyl or cyclohex-2-enyl group.

The expression heterocycloalkyl refers to a cycloalkyl group as defined above in which one or more (preferably 1, 2 or 3) ring carbon atoms have been replaced by an oxygen, nitrogen, silicon, selenium, phosphorus or sulphur atom (preferably oxygen, sulphur or nitrogen). A heterocycloalkyl group has preferably 1 or 2 ring(s) containing from 3 to 10 (especially 3, 4, 5, 6 or 7) ring atoms. The expression heterocycloalkyl refers furthermore to groups in which one or more hydrogen atoms have been replaced by fluorine, chlorine, bromine or iodine atoms or by OH, =O, SH, =S, NH₂, =NH or NO₂ groups. Examples are a piperidyl, piperazinyl, morpholinyl, urotropinyl, pyrrolidinyl, tetrahydrothiophenyl, tetrahydropyranyl, tetrahydrofuryl or 2-pyrazolinyl group and also lactams, lactones, cyclic imides and cyclic anhydrides.

The expression alkylcycloalkyl refers to groups containing both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings containing from 3 to 10 (especially 3, 4, 5, 6 or 7) carbon atoms, and one or two alkyl, alkenyl or alkynyl groups having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, selenium, phosphorus or sulphur atom (preferably oxygen, sulphur or nitrogen). A heteroalkylcycloalkyl group preferably contains 1 or 2 ring systems having from 3 to 10 (especially 3, 4, 5, 6 or 7) ring atoms, and one or two alkyl, alkenyl, alkynyl or heteroalkyl groups having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that has one or more rings containing from 6 to 50 carbon atoms, preferably 6 to 14, further preferably from 6 to 10 (especially 6) carbon atoms. The expression aryl refers furthermore to groups in which one or more hydrogen atoms have been replaced by fluorine, chlorine, bromine or iodine atoms or by OH, SH, NH₂ or NO₂ groups. Examples are a phenyl, naphthyl, biphenyl, 2-fluorophenyl, anilinyl, 3-nitrophenyl or 4-hydroxyphenyl group.

The expression heteroaryl refers to an aromatic group that has one or more rings containing from 5 to 50 ring atoms, preferably 5 to 14, further preferably from 5 to 10 (especially 5 or 6) ring atoms, and contains one or more (preferably 1, 2, 3 or 4) oxygen, nitrogen, phosphorus or sulphur ring atoms (preferably O, S or N). The expression heteroaryl refers furthermore to groups in which one or more hydrogen atoms have been replaced by fluorine, chlorine, bromine or iodine atoms or by OH, SH, NH₂ or NO₂ groups. Examples are 4-pyridyl, 2-imidazolyl, 3-phenylpyrrolyl, thiazolyl, oxazolyl, triazolyl, tetrazolyl, isoxazolyl, indazolyl, indolyl, benzimidazolyl, pyridazinyl, quinolinyl, purinyl, carbazolyl, acridinyl, pyrimidyl, 2,3'-bifuryl, 3-pyrazolyl and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylarylcycloalkyl and alkylarylcycloalkenyl groups. Specific examples of aralkyls are toluene, xylene, mesitylene, styrene, benzyl chloride, o-fluorotoluene, 1H-indene, tetralin, dihydronaphthalene, indanone, phenylcyclopentyl, cumene, cyclohexylphenyl, fluorene and indan. An aralkyl group preferably contains one or two aromatic ring systems (1 or 2 rings) containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to an aralkyl group as defined above in which one or more (preferably 1, 2, 3 or 4) carbon atoms have been replaced by an oxygen, nitrogen, silicon, selenium, phosphorus, boron or sulphur atom (preferably oxygen, sulphur or nitrogen), that is to say to groups containing both aryl or heteroaryl and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (1 or 2 rings) containing from 5 or 6 to 10 carbon atoms and one or two alkyl, alkenyl and/or alkynyl groups containing 1 or 2 to 6 carbon atoms and/or a cycloalkyl group containing 5 or 6 ring carbon atoms, 1, 2, 3 or 4 of those carbon atoms having been replaced by oxygen, sulphur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroarylheterocycloalkyl, heteroarylheterocycloalkenyl, heteroarylalkylcycloalkyl, heteroarylalkylheterocycloalkenyl, heteroarylheteroalkylcycloalkyl, heteroarylheteroalkylcycloalkenyl and heteroarylheteroalkylheterocycloalkyl groups, the cyclic groups being saturated or mono-, di- or tri-unsaturated. Specific examples are a tetrahydroisoquinolinyl, benzoyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups in which one or more hydrogen atoms of such groups have been replaced by fluorine, chlorine, bromine or iodine atoms or by OH, =O, SH, =S, NH₂, =NH or NO₂ groups.

Furthermore, preferably all alkyl, alkenyl, alkynyl, heteroalkyl, cycloalkyl, heterocycloalkyl, alkylcycloalkyl, heteroalkylcycloalkyl, aryl, heteroaryl, aralkyl and heteroaralkyl groups as defined herein may be optionally substituted.

The expression "optionally substituted" refers to groups in which one or more hydrogen atoms optionally have been replaced by fluorine, chlorine, bromine or iodine atoms or by OH, =O, SH, =S, NH₂, =NH or NO₂ groups. This expression refers furthermore to groups that are optionally substituted by one or more (e.g. 1, 2 or 3) unsubstituted C₁-C₆alkyl, C₂-C₆alkenyl, C₂-C₆alkynyl, C₁-C₆heteroalkyl, C₃-C₁₀cycloalkyl, C₂-C₉heterocycloalkyl, C₆-C₁₀aryl, C₁-C₉heteroaryl, C₇-C₁₂aralkyl or C₂-C₁₁heteroaralkyl groups.

Owing to their substitution, compounds of formula (I) may contain one or more centres of chirality. The present invention therefore includes both all pure enantiomers and all pure diastereoisomers and also mixtures thereof in any mixing ratio. The present invention moreover also includes all cis/trans-isomers of the compounds of the general formula (I) and also mixtures thereof. The present invention moreover includes all tautomeric forms of the compounds of formula (I).

Preferably, X is O or NH, especially preferably, X is O.

Further preferably, R⁹ is methyl.

Further preferably, R¹⁰ is H or methyl, especially methyl.

Moreover preferably, R⁵, R⁶ and R⁷ are independently hydrogen or C₁₋₄ alkyl, especially preferably hydrogen or methyl.

Further preferably, R⁵ is hydrogen.

Further preferably, R⁶ is methyl.

Further preferably, R⁷ is methyl.

Further preferably, R⁴ and R⁸ together are a group of formula -(CH₂)ₙ- wherein this group may optionally be substituted (e.g. by a hydroxy group) and n is 2, 3, 4 or 5.

Especially preferably, R⁴ and R⁸ together are a group of formula -(CH₂)₃-.

Further preferably, R² is hydrogen.

Further preferably, R³ is a C₁-₆ alkyl group.

Especially preferably, R³ is a group of formula -CH₂CH(CH₃)₂.

Further preferably, R¹ is a group of formula -CH₂Ar wherein Ar is an optionally substitured aryl or heteroaryl group. Especially preferably, Ar is a phenyl group.

Further preferably, R¹, R², R³ and/or R⁴ are independently selected from H, CH₃, CH₂OH or the following groups: 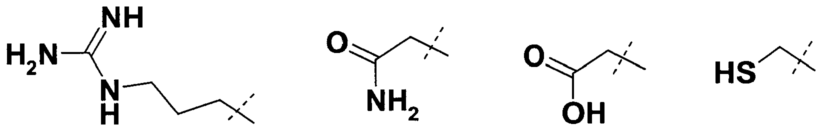 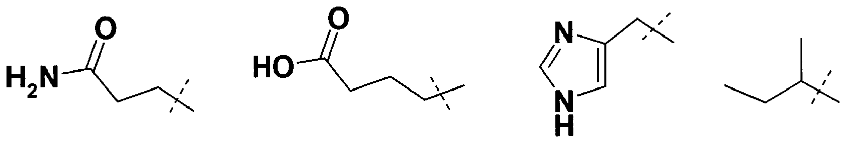 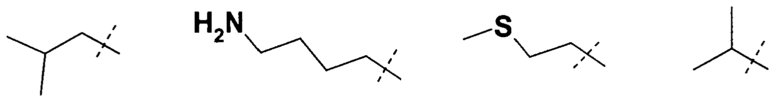 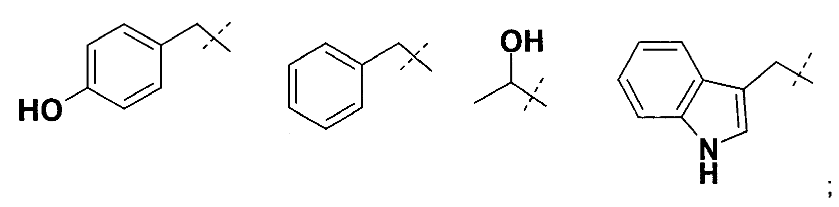 or
R¹ and R⁵, R² and R⁶, R³ and R⁷ and/or R⁴ and R⁸ together are a group of formula -(CH₂)₃-.

Further preferred are compounds of formula (II): 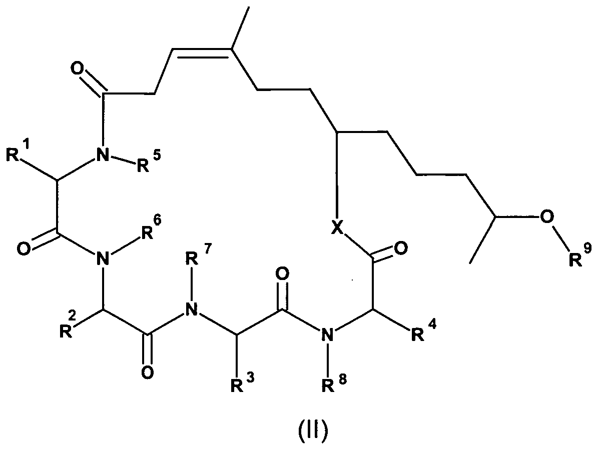 wherein R¹, R², R³, R⁴, R⁵, R⁶, R⁷ R⁸, R⁹ and X are as defined above.

Especially preferred, the compound of formula (I) or (II) has the following structure (Haprolid):

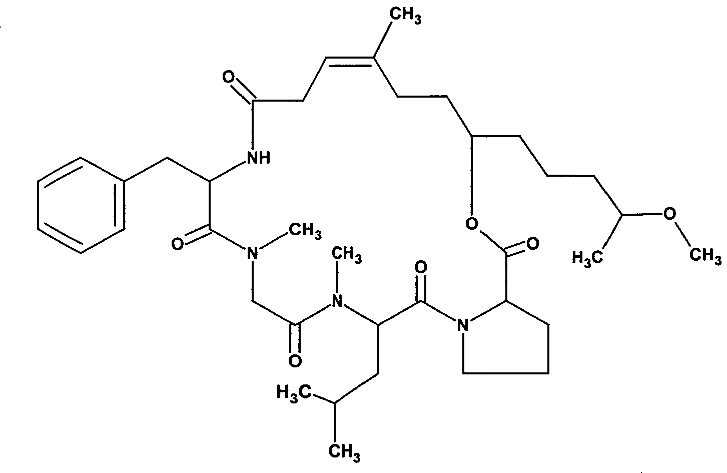

The therapeutic use of compounds of formula (I) or (II), their pharmacologically acceptable salts or solvates and hydrates and also formulations and pharmaceutical compositions also lie within the scope of the present invention, especially, the use of compounds of formula (I) or (II), their pharmacologically acceptable salts or solvates and hydrates and also formulations and pharmaceutical compositions as antiviral agents, especially for the treatment of hepatitis C as well as their use for the preparation of medicaments for the treatment of viral infections, especially for the treatment of hepatitis C. Furthermore, the present invention relates to compounds and/or pharmaceutical compositions as described herein for use in the treatment of viral infections, especially for use in the treatment of hepatitis C.

The pharmaceutical compositions according to the present invention comprise at least one compound of formula (I) or (II) as active ingredient and, optionally, carrier substances and/or adjuvants. Further, these pharmaceutically compositions may comprise other antiviral ingredients.

Examples of pharmacologically acceptable salts of the compounds of formula (I) or (II) are salts of physiologically acceptable mineral acids, such as hydrochloric acid, sulphuric acid and phosphoric acid, or salts of organic acids, such as methanesulphonic acid, p-toluenesulphonic acid, lactic acid, acetic acid, trifluoroacetic acid, citric acid, succinic acid, fumaric acid, maleic acid and salicylic acid. Further examples of pharmacologically acceptable salts of the compounds of formula (I) or (II) are alkali metal and alkaline earth metal salts such as, for example, sodium, potassium, lithium, calcium or magnesium salts, ammonium salts or salts of organic bases such as, for example, methylamine, dimethylamine, triethylamine, piperidine, ethylenediamine, lysine, choline hydroxide, meglumine, morpholine or arginine salts. Compounds of formula (I) or (II) may be solvated, especially hydrated. The hydration may take place, for example, during the preparation process or as a consequence of the hygroscopic nature of the initially anhydrous compounds of formula (I) or (II). When the compounds of formula (I) or (II) comprise asymmetric C-atoms, they may be present either in the form of achiral compounds, diastereoisomeric mixtures, mixtures of enantiomers or in the form of optically pure compounds.

The pro-drugs to which the present invention also relates comprise a compound of formula (I) or (II) and at least one pharmacologically acceptable protecting group which will be removed under physiological conditions, such as, for example, an alkoxy-, aralkyloxy-, acyl- or acyloxy group, such as, for example, an ethoxy, benzyloxy, acetyl or acetyloxy group.

The present invention relates also to the use of those active ingredients in the preparation of medicaments (especially antiviral drugs). In general, compounds of formula (I) or (II) are administered either individually, or in combination with any other desired therapeutic agent, using the known and acceptable methods. Such therapeutically useful agents may be administered, for example, by one of the following routes: orally, for example in the form of dragées, coated tablets, pills, semisolid substances, soft or hard capsules, solutions, emulsions or suspensions; parenterally, for example in the form of an injectable solution; rectally in the form of suppositories; by inhalation, for example in the form of a powder formulation or a spray; transdermally or intranasally. For the preparation of such tablets, pills, semisolid substances, coated tablets, dragées and hard gelatine capsules, the therapeutically usable product may be mixed with pharmacologically inert, inorganic or organic pharmaceutical carrier substances, for example with lactose, sucrose, glucose, gelatine, malt, silica gel, starch or derivatives thereof, talcum, stearic acid or salts thereof, skimmed milk powder, and the like. For the preparation of soft capsules, pharmaceutical carrier substances such as, for example, vegetable oils, petroleum, animal or synthetic oils, wax, fat and polyols may be used. For the preparation of liquid solutions and syrups, pharmaceutical carrier substances such as, for example, water, alcohols, aqueous saline solution, aqueous dextrose, polyols, glycerol, vegetable oils, petroleum and animal or synthetic oils may be used. For suppositories, pharmaceutical carrier substances such as, for example, vegetable oils, petroleum, animal or synthetic oils, wax, fat and polyols may be used. For aerosol formulations, compressed gases that are suitable for this purpose, such as, for example, oxygen, nitrogen and carbon dioxide may be used. The pharmaceutically acceptable agents may also comprise additives for preserving and stabilising, emulsifiers, sweeteners, flavourings, salts for altering the osmotic pressure, buffers, encapsulation additives and antioxidants.

Combinations with other therapeutic agents may comprise other antiviral agents, especially other inhibitors of hepatitis C Virus (HCV).

For the prevention and/or treatment of viral infections such as hepatitis C, the dose of the biologically active compound according to the invention may vary within wide limits and may be adjusted to individual requirements. Generally, a dose of from 10 mg to 4000 mg per day is suitable, a preferred dose being from 50 to 3000 mg per day. In suitable cases, the dose may also be below or above the stated values. The daily dose may be administered as a single dose or in a plurality of doses. A typical individual dose contains approximately 50 mg, 100 mg, 250 mg, 500 mg, 1 g or 2 g of the active ingredient.

### EXAMPLE

### Isolation of Haprolid:

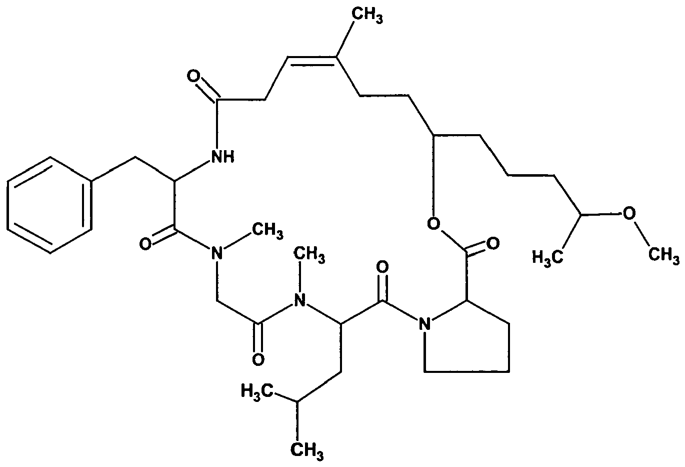

A fermentation (90L) of *Byssovorax cruenta*, strain Har1 (The strain has been deposited at the German Collection of Microorganisms and Cell Cultures (DSMZ) Braunschweig, Germany under the accession number DSM 14567), was performed in presence of 0.9 L Amberlite XAD 16. At the end of the fermentation, the resin was harvested by filtration with a process filter (210 µm mesh), washed with water to remove adherent cells, and extracted with 5 L of acetone to yield raw haprolid. After evaporation of the acetone, the residual water layer was extracted two times with ethyl acetate. The organic layer was dried, filtrated and concentrated in vacuo to give a crude extract (yield 7.9 g).

Initial separation was achieved by silica gel column chromatography (130 g of silica gel Merck 60 15-40 µm) with the eluents: 1. ethyl acetate/n-heptane 2:1 (0.9 L); 2. ethyl acetate (0.9 L); 3. ethyl acetate/methanol 8:2 (0.7 L); 4. ethyl acetate/methanol 1:1 (0.6 L). Based on TLC analyses the chromatography gave 4 fractions. The third fraction (0.73 g) was further separated [column 30×480 mm, Kronlab ODS-AQ 120 16 µm, solvent: acetonitrile/water 55:45, flow rate: 18 mL/min, UV detection 206 nm]. After evaporation of the organic solvent from the haprolid containing fraction, the remaining water was extracted with ethyl acetate to yield 116 mg pure haprolid.

Physico-chemical characterisation of Haprolid:
Formula: C₃₈H₅₈N₄O₇
Molecular weight: 682.9 g/mol
HR ESI MS: [M+H]⁺ calcd.: 683.4378 found: 683.4383
IR (KBr): v = 2957, 2925,2870, 1738, 1650,1452, 1193 cm⁻¹.
UV (MeOH): λₘₐₓ (Ig ε) = 205 (4.49), 220 (sh) nm.
TLC (silica gel): solvent dichloromethane:methanol 9:1, *R*_{f} = 0.75
HPLC: column 2×125 mm, Nucleodur Gravity 120 5µm C18 (Macherey Nagel),
   solvent A: 95/5 water/acetonitrile + 5 mM NH₄Ac, pH: 5.5
   solvent B: 5/95 water/acetonitrile + 5 mM NH₄Ac, pH: 5.5
   55% B to 60% B in 10 min, 60% B isocratic
   flow 0.3 mL/min,
   detection UV 200-400 nm
   *R*ₜ = 5.8 min.

**Table 1: NMR-Data of Haprolid in DMSO-d₆**

| phenylalanine | | | | | |
|---|---|---|---|---|---|
| proton | δ (ppm) | m | J [Hz] | carbon | δ (ppm) |
| | | | | C-1 | 169.9 |
| 2-H | 4.87 | ddd | 9.6, 9.6, 5.4 | C-2 | 49.6 |
| 3-H_{b} | 3.04 | dd | 13.8, 5.4 | C-3 | 37.5 |
| 3-Hₐ | 2.75 | dd | 13.9, 4.2 | | |
| | | | | C-4 | 138.1 |
| 5,6-H | 7.2 | m | | C-5,6 | 129.3 |
| 7,8-H | 7.24 | m | | C-7,8 | 127.4 |
| 9-H | 7.19 | m | | C-9 | 125.4 |
| 2-NH | 8.28 | d | 10.0 | | |

| sarkosine | | | | | |
|---|---|---|---|---|---|
| | | | | C-1 | 168.3 |
| 2-H_{b} | 4.75 | d | 17.6 | C-2 | 50.2 |
| 2-Hₐ | 3.83 | d | 17.6 | | |
| 2-NH₃ | 2.81 | s | | N-CH₃ | 34.7 |

| *N*-methyl-leucine | | | | | |
|---|---|---|---|---|---|
| | | | | C-1 | 169.6 |
| 2-H | 5.32 | dd | 11.4, 3.8 | C-2 | 51.2 |
| 3-H_{b} | 1.74-1.66 | m | | C-3 | 36.5 |
| 3-Hₐ | 1.39-1.32 | m | | | |
| 4-H | 1.48-1.38 | m | | C-4 | 24.2 |
| 5-H₃ | 0.86 | d | 6.5 | C-5 | 20.7 |
| 6-H₃ | 0.91 | d | 6.5 | C-6 | 22.7 |
| 2-NH₃ | 2.84 | s | | N-CH₃ | 29.1 |
| proline | | | | | |
| 2 - H | 4.23 | d | 8.8, 2.8 | C-1 | 171.9 |
| 3 - H_{b} | 2.16-2.11 | m | | C-2 | 58.4 |
| 3 - Hₐ | 1.80-1.76 | m | | C-3 | 28.4 |
| 4 - H_{b} | 2.02-1.96 | m | | C-4 | 24.0 |
| 4 - Hₐ | 2.02-1.96 | m | | C-5 | 46.0 |
| 5 - H_{b} | 3.64-3.59 | m | | | |
| 5 - Hₐ | 3.55 | ddd | 9.0, 9.0, 7.6 | | |

| substituted unsaturated carboxylic acid | | | | | |
|---|---|---|---|---|---|
| proton | δ (ppm) | m | J [Hz] | carbon | δ (ppm) |
| | | | | C-1 | 170.1 |
| 2-H_{b} | 2.64 | dd | 14.1, 11.1 | C-2 | 35.2 |
| 2-Hₐ | 2.29 | dbr. | 14.3 | | |
| 3-H | 4.52 | dbr. | 9.7 | C-3 | 121.1 |
| | | | | C-4 | 134.2 |
| 5-H_{b} | 2.20-2.14 | m | | C-5 | 26.4 |
| 5-Ha | 1.73-1.69 | m | | | |
| 6-H_{b} | 1.64-1.56 | m | | C-6 | 30.9 |
| 6-Hₐ | 1.64-1.56 | m | | | |
| 7-H | 4.62 | m | | C-7 | 72.2 |
| 8-H_{b} | 1.46-1.40 | m | | C-8 | 34.5 |
| 8-Ha | 1.46-1.40 | m | | | |
| 9-H_{b} | 1.40-1.20 | m | | C-9 | 20.2 |
| 9-Hₐ | 1.40-1.20 | m | | | |
| 10-H_{b} | 1.40-1.29 | m | | C-10 | 35.1 |
| 10-Hₐ | 1.40-1.29 | m | | | |
| 11-H | 3.23 | m | | C-11 | 75.4 |
| 4-Me | 1.52 | s | | C-4 Me | 22.2 |
| 11-Me | 1.03 | d | 6.2 | C-11 Me | 18.5 |
| 11-OMe | 3.19 | s | | C-11 OMe | 54.8 |

### Cell-based assay for screening the complete HCV replication cycle (4):

To identify novel bioactive compounds with HCV-specific antiviral activity, a robust and sensitive cell based assay based on a cell line derived from Huh-7 human hepatocarcinoma cells has been developed. The cells used in this assay originate from Huh7-Lunet cells that are highly permissive to HCV RNA replication (1), but which express only limited quantities of CD81, an essential HCV entry factor (2). Robust expression of human CD81 was established using lentiviral gene transfer thus rendering these cells also highly susceptible to HCV cell entry (3). Further, a lentiviral vector has been employed to introduce a constitutively expressed transgene for a secreted gaussia luciferase (G-Luc) from the marine copepod *Gaussia princeps*. The resulting cell line was designated Huh7-Lunet-hCD81-Gluc and is highly permissive for HCV propagation. In addition it expresses a simple biomarker (G-Luc) which reflects both cell number and cell viability and that can be monitored non-invasively by sampling the culture fluid. To quantify HCV replication in these cells, the highly efficient HCV reporter virus Luc-Jc1 has been utilized which expresses the firefly luciferase derived from *Photinus pyralis* (2). As a consequence, cell viability and HCV replication can be measured simultaneously using a dual luciferase assay determining G-Luc and F-Luc activities in culture fluids and cell lysates, respectively.

To permit simple detection of influences on RNA replication, virus production and also cell entry, a screening protocol has been developed that encompasses the entire HCV replication cycle and that is based on two measurements of HCV-dependent F-Luc activity: First, Huh7-Lunet-hCD81-Gluc cells are transfected with Luc-Jc1 RNA and seeded into culture plates. Four hours later, individual compounds are added to the culture fluid and co-cultured with the HCV-replicating cells throughout the entire assay. A first reading of F-Luc activity is conducted 48 h post transfection and reflects HCV RNA translation and replication efficiency in the presence of compounds since at this time point secondary rounds of infection do not significantly contribute to the HCV-specific F-Luc signal (2). In parallel, culture fluid of these cells containing infectious reporter viruses produced at this time point as well as the compounds are transferred to naive Huh7-Lunet-hCD81-Gluc cells. Another 48 h later, both G-Luc and F-Luc activities produced in the inoculated cells are determined. These readings reflect cell viability and the efficiency of HCV RNA replication, virus production and cell entry, thus encompassing at least one entire replication cycle of HCV in the presence of compounds.

References:
1. Friebe, P., Boudet, J., Simorre, J. P., and Bartenschlager, R. 2005. Kissing-loop interaction in the 3' end oft he hepatitis C virus genome essential for RNA replication. J. Virol. 79: 380-392.
2. Koutsoudakis, G., Kaul, A., Steinmann, E., Kallis, S. Lohmann, V., Pietschmann, T., and Bartenschlager, R. 2006. Characterization of the early steps of hepatitis C virus infection by using luciferase reporter viruses. J. Virol. 80: 5308-5320.
3. Bitzegeio, J., Bankwitz, D., Hueging, K., Haid, S., Brohm, C., Zeisel, M.B., Herrmann, E., Iken, M., Ott, M., Baumert, T.F., and Pietschmann, T. 2010. Adaptation of hepatitis C virus to mouse CD81 permits infection of mouse cells in the absence of human entry factors. PLoS Pathog. 6:e1000978.
4. Gentzsch, J., Hinkelmann, B., Kaderali, L., Irschik, H., Jansen, R., Sasse, F., Frank, R., and Pietschmann, T. 2011. Hepatitis C virus complete life cycle screen for identification of small molecules with pro- or antiviral activity. Antiviral Res. 89 (2) : 136-48.

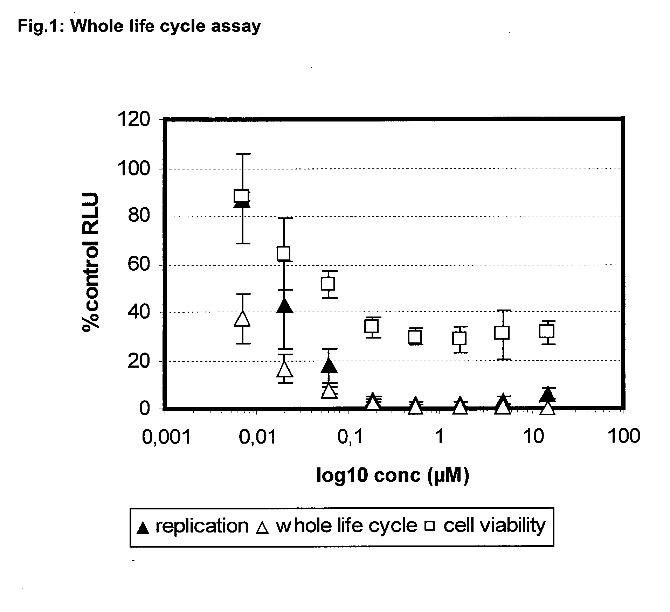

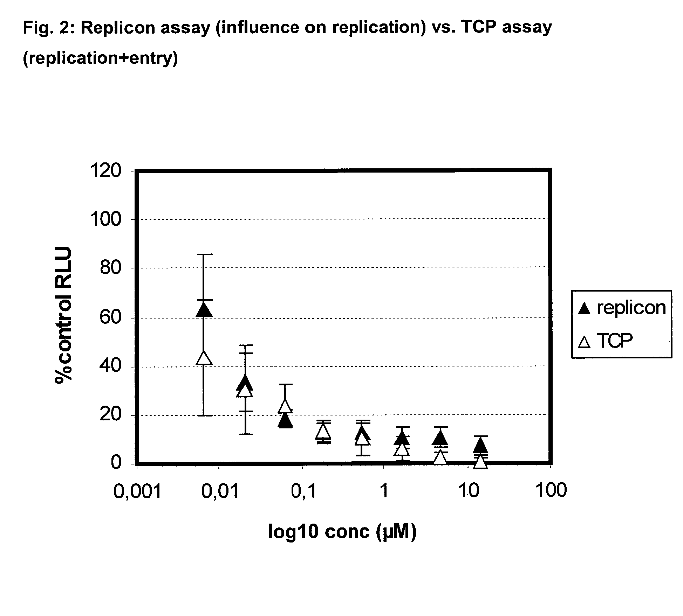

Haprolid has been identified as a potent inhibitor of HCV. Experiments confirmed its antiviral activity at nanomolar concentrations (EC50 < 6nM) with toxicity in low micromolar ranges (TC50 - 0.1 µM) (Fig. 1). The compound displayed a moderate influence on HCV RNA replication (5-fold reduction at non-toxic concentrations, Fig. 2) as shown with the subgenomic HCV replicon system, and a similar influence when analyzed with HCV_{TCP}. Employing HCV_{TCP}, influences on HCV RNA replication and virus entry are quantified. Since no increased susceptibility of HCV_{TCP} to the drug compared to subgenomic replicons has been observed, these data indicate that the drug primarily affects HCV RNA replication but not the HCV cell entry steps (Fig. 2).

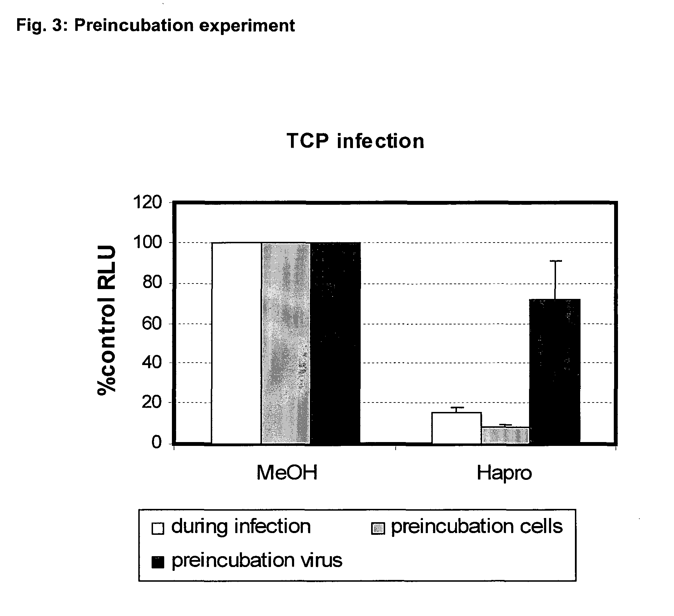

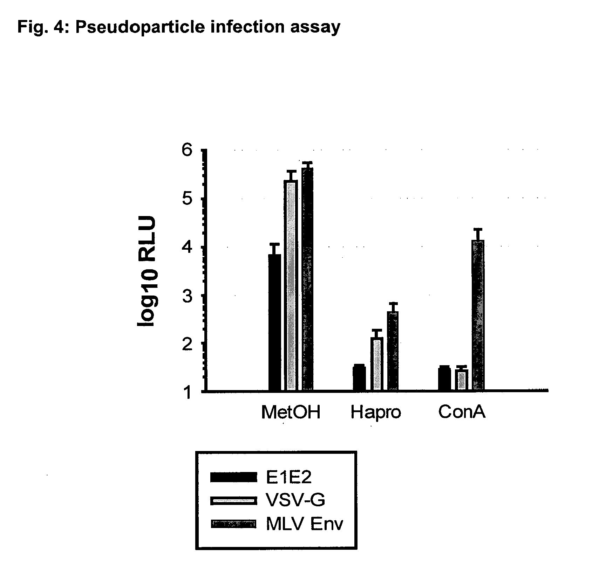

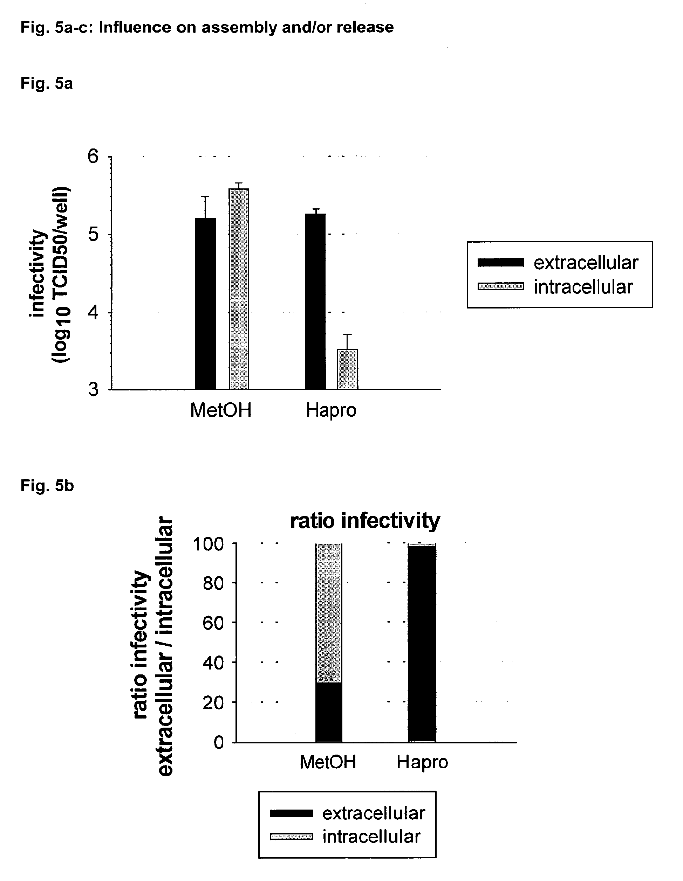

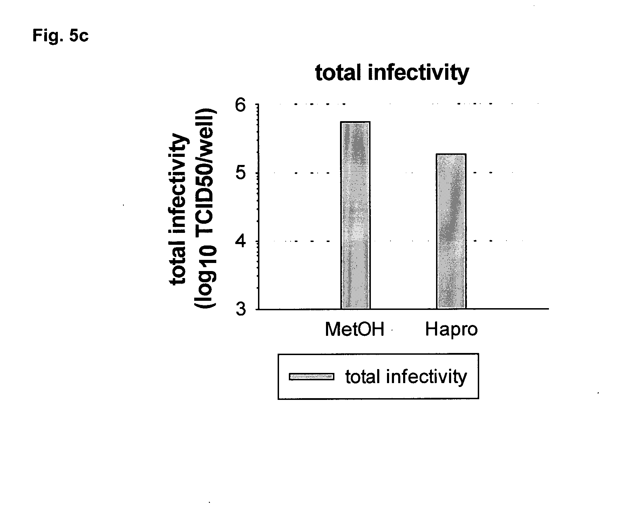

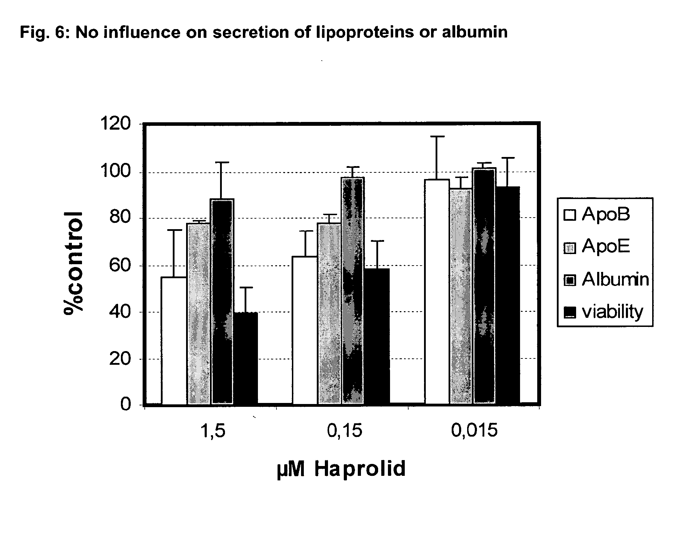

Adding the compound to retroviral particles pseudotyped with the glycoproteins of either HCV, VSV (vesicular stomatitis virus) or MLV (murine leukemia virus), another method of testing for viral entry, resulted in a strong decrease of infectivity (100- to 1000-fold, Fig. 4). These results indicate that Haprolid interferes with retroviral cell entry steps in a viral glycoprotein-independent fashion possible blocking steps like e.g. trafficking of retroviral cores, reverse transcription, nuclear import, or integration. Moreover, an influence of the compound on the steps leading to assembly and release of infectious HCV particles has been shown. Total production of infectious virus particles was reduced about 3-fold (Fig. 5c). Addition of the compound led to a 100-fold reduction of intracellular virus titer (Fig. 5a), indicating that processes determining export of virus particles from infected cells are modulated by the drug. However, this was not caused by an influence of the drug on lipoprotein secretion (ApoB/E, Fig. 6), on which HCV secretion strongly depends, nor a general influence on the secretory capacity of the cell as shown for albumin (Fig. 6). Finally, preincubation experiments utilizing the HCV_{TCP} system revealed an influence of the compound on the cell rather than on viral component since only preincubation of the cells, but not of the virus resulted in a comparable effect as addition of compound during infection (Fig. 3).

In conclusion, a novel, potent HCV inhibitor has been identified which affects assembly and release of particles as well as HCV replication. Additionally, it interferes with early steps of the retroviral life cylce indicating a broader scope of antiviral activity. This inhibitor influences the host cell rather than the virus itself which could be beneficial in the prevention of resistance mutations which often occur during HCV infection.
Figures 1 to 6 show a summary of the the influence of Haprolid on HCV: Figure 1 shows the results of a whole life cycle assay.
Figure 2 shows the results of a replicon assay (influence on replication) vs. TCP assay (replication+entry).
Figure 3 shows the results of a preincubation experiment in the context of HCV TCP infection.
Figure 4 shows the results of a pseudoparticle infection assay.
Figure 5 shows the influence on assembly and/or release.
Figure 6 shows that there is no influence on secretion of lipoproteins or albumin.

## Claims

1. Compound of general formula (I): 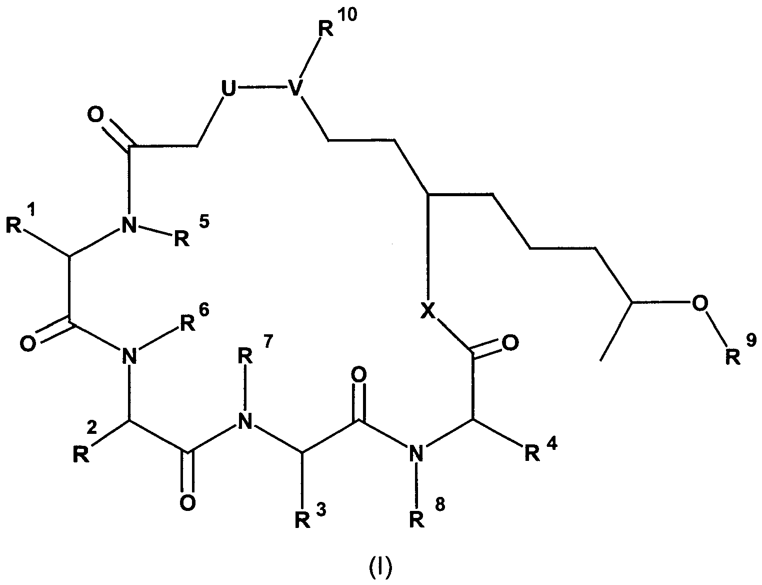 wherein
X is O, S or NR¹¹;
U-V are selected from the following groups: 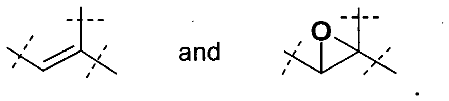 R¹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R² is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R³ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁴ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁵ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁶ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁷ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁸ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R⁹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group;
R¹⁰ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group; and
R¹¹ is a hydrogen atom, an alkyl, an alkenyl, an alkynyl, a heteroalkyl, a cycloalkyl, a heterocycloalkyl, an alkylcycloalkyl, a heteroalkylcycloalkyl, an aryl, a heteroaryl, an aralkyl or a heteroaralkyl group; or
R¹ and R⁵ and/or R¹ and R⁶ and/or R⁵ and R⁶ and/or R¹ and R² and/or R² and R⁶ and/or R² and R⁷ and/or R⁶ and R⁷ and/or R² and R³ and/or R³ and R⁷ and/or R³ and R⁸ and/or R⁷ and R⁸ and/or R³ and R⁴ and/or R⁴ and R⁸ and/or R⁴ and R¹¹ and/or R⁸ and R¹¹ together are part of an optionally substituted heterocycloalkyl group;
or a pharmacologically acceptable salt, solvate, hydrate or a pharmacologically acceptable formulation thereof.

2. Compound according to claim 1, having the following general formula (II):

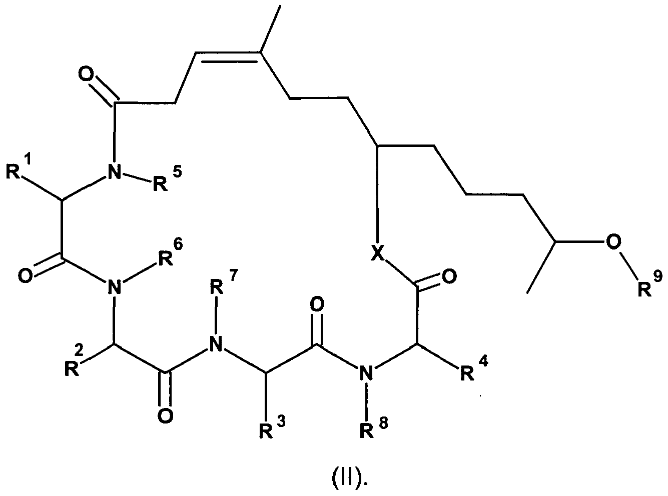

3. Compound according to claim 1 or 2, wherein X is O.

4. Compound according to anyone of the preceding claims, wherein R⁹ is methyl.

5. Compound according to anyone of claims 1, 3 or 4, wherein R¹⁰ is methyl.

6. Compound according to anyone of the preceding claims, wherein R⁵, R⁶ and R⁷ are independently hydrogen or C₁₋₄ alkyl.

7. Compound according to anyone of the preceding claims, wherein R⁴ and R⁸ together are a group of formula -(CH₂)ₙ- wherein this group may optionally be substituted and n is 2, 3, 4 or 5.

8. Compound according to anyone of the preceding claims, wherein R² is hydrogen.

9. Compound according to anyone of the preceding claims, wherein R³ is a C₁-₆ alkyl group.

10. Compound according to anyone of the preceding claims, wherein R¹ is a group of formula -CH₂Ar wherein Ar is an optionally substitured aryl or heteroaryl group.

11. Compound according to claim 1 having the following structure:

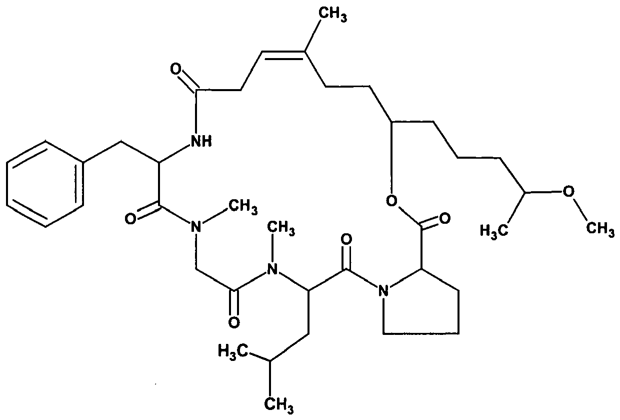

12. Compound (Haprolid) having the following parameters:
Formula: C₃₈H₅₈N₄O₇;
Molecular weight: 682.9 g/mol;
HR ESI MS: [M+H]⁺ calcd.: 683.4378 found: 683.4383;
IR (KBr): v = 2957, 2925, 2870, 1738, 1650, 1452, 1193 cm⁻¹;
UV (MeOH): λₘₐₓ (Ig ε) = 205 (4.49), 220 (sh) nm;
TLC (silica gel): solvent dichloromethane:methanol 9:1, *R*_{f} = 0.75;
HPLC: column 2x125 mm, Nucleodur Gravity 120 5µm C18 (Macherey
Nagel),
solvent A: 95/5 water/acetonitrile + 5 mM NH₄Ac, pH: 5.5 solvent B: 5/95 water/acetonitrile + 5 mM NH₄Ac, pH: 5.5 55% B to 60% B in 10 min, 60% B isocratic
flow 0.3 mL/min,
detection UV 200-400 nm
*R*ₜ = 5.8 min;
and the NMR Data shown in Table 1.

13. Pharmaceutical composition comprising a compound according to anyone of the preceding claims and optionally one or more carrier substances and/or one or more adjuvants.

14. Compound or pharmaceutical composition according to anyone of the preceding claims for use in the treatment of viral infections.

15. A method of producing a compound according to anyone of claims 1 to 12 **characterized in that** the *Byssovorax cruenta*, strain Har1 is used for the production.
